(19) Europäisches Patentamt
European Patent Office
Office européen des brevets

(11) Veröffentlichungsnummer: **0 492 257 A1**

(12) # EUROPÄISCHE PATENTANMELDUNG

(21) Anmeldenummer: **91121108.4**

(22) Anmeldetag: **09.12.91**

(51) Int. Cl.[5]: **C07D 417/04**, A61K 31/54, //(C07D417/04,285:00,215:00)

(30) Priorität: **21.12.90 DE 4041074**

(43) Veröffentlichungstag der Anmeldung: **01.07.92 Patentblatt 92/27**

(84) Benannte Vertragsstaaten: **AT BE CH DE DK ES FR GB GR IT LI LU NL SE**

(71) Anmelder: **MERCK PATENT GESELLSCHAFT MIT BESCHRÄNKTER HAFTUNG**
**Frankfurter Strasse 250**
**W-6100 Darmstadt(DE)**

(72) Erfinder: **Jonas, Rochus, Dr.**
**Kesselhutweg 12**
**W-6100 Darmstadt(DE)**
Erfinder: **Lues, Ingeborg, Dr.**
**Katharinenstrasse 2**
**W-6100 Darmstadt(DE)**
Erfinder: **Beier, Norbert, Dr.**
**Georgenhäuser-Strasse 19**
**W-6107 Reinheim 5(DE)**
Erfinder: **Klockow, Michael, Dr.**
**Steinacker 12**
**W-6101 Rossdorf(DE)**
Erfinder: **Minck, Klaus-Otto, Dr.**
**Büchestrasse 8**
**W-6105 Ober-Ramstadt(DE)**

(54) **Thiadiazinonderivate.**

(57) Thiadiazinone der Formel I

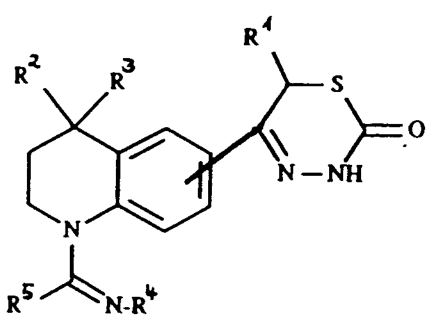

I

worin
$R^1$, $R^2$, $R^3$, $R^4$ und $R^5$ die in Anspruch 1 angegebene Bedeutung haben, zeigen positiv-inotrope sowie vasodilatierende Wirkung und eignen sich zur Bekämpfung von kardiovaskulären Erkrankungen.

EP 0 492 257 A1

Die Erfindung betrifft neue Thiadiazinonderivate der Formel I

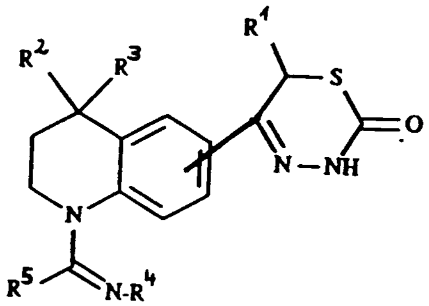

I

worin

| | |
|---|---|
| $R^1$, $R^2$ und $R^3$ | jeweils H oder A, |
| $R^4$ | H, A, Ar, Ar-alkyl oder Cycloalkyl mit 3-7 C-Atomen, |
| $R^5$ | A, Ar oder Cycloalkyl mit 3-7 C-Atomen, |
| A | Alkyl mit 1-8 C-Atomen, |
| Ar | einen unsubstituierten oder einen ein-, zwei- oder dreifach durch A, OH, OA, $SR^6$, $SOR^6$, $SO_2R^6$, Hal, $NH_2$, NHA, $NA_2$, $NO_2$, $O-CH_2-O$ oder CO-A substituierten Phenylrest, |
| "-alkyl" | Alkylen mit 1-5 C-Atomen, |
| $R^6$ | H oder Ar und |
| Hal | F, Cl, Br oder I bedeuten |

sowie deren Salze.

Thiadiazinonderivate, die der Formel I entsprechen, deren Stickstoffatom des Chinolinrestes jedoch durch ein H-Atom oder einen Acylrest substituiert ist, sind aus EP 294 647 bzw. aus DE 37 19 031 A1 bekannt.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I bei guter Verträglichkeit wertvolle pharmakologische Eigenschaften besitzen. Insbesondere zeigen sie eine Wirkung auf die Herzkraft (positiv inotrope Wirksamkeit); ferner wirken die Substanzen vasodilatierend und fördern daher die Durchblutung. Die vasodilatierende und die Herzwirkung kann z.B. an narkotisierten oder wachen Hunden, Katzen, Affen oder Minischweinen, die positiv inotrope Wirkung auch an isolierten Herzpräparationen (z.B. Vorhof, Papillarmuskel oder perfundiertes Ganzherz) von Ratte, Meerschweinchen, Katze oder Hund ermittelt werden, z.B. nach Methoden, wie sie in Arzneimittelforschung, Band 31 (I) Nr. 1a (1981), Seiten 141 bis 170, oder von Schliep et al. im 9th International Congress of Pharmacol., London, Abstracts of papers 9P, beschrieben sind.

Überdies zeichnen sich die Verbindungen dadurch aus, daß sie eine Steigerung der Ca-Empfindlichkeit der kontraktilen Proteine bewirken und geringere Nebenwirkungen, wie z.B. Arrhythmien, im Vergleich zu anderen positiv inotrop wirkenden Substanzen hervorrufen.

Weiterhin treten antithrombotische, thrombozytenaggregationshemmende und die Erythrozytenform beeinflussende Eigenschaften auf. Die Beeinflussung der Thrombozytenfunktion im Sinne einer Aggregationshemmung kann an der Ratte ex vivo im Test nach Born (Nature 194, 927-929, 1962) nachgewiesen werden. Die antithrombotische Wirkung zeigt sich in der Verlängerung der Blutungszeit nach Stella (Thrombos. Res. 7, 709-716, 1975) in der Verminderung des Thrombusgewichtes bei der kälteinduzierten Thrombosierung der Jugular-Vene bei der Ratte nach Meng (Ther. Ber. 47, 69-79, 1975) und der Erhöhung der zur vollständigen Thrombosierung notwendigen Laserimpulse an der Mensenterial-Venole der Ratte, entsprechend einer Abwandlung der Methode nach Kovacs (Microvasc. Res. 6, 194-201, 1973).

Die günstige Wirkung auf die Erythrozytenverformbarkeit ist im Nucleoporefilter nach Schmid-Schönbein (Pflüger's Archiv 338, 93-114, 1973) nachweisbar. Auch lassen sich günstige Effekte auf die Fibrinolyse/Euglobulinlysiszeit nach v. Kaulla (Progr. Chem. Fibrinol., Thrombol. 1, 131-149, 1975; ed J.F. Davidson, Raven Press, N.Y.) feststellen.

Die Verbindungen können daher als Arzneimittelwirkstoffe in der Human- und Veterinärmedizin verwendet werden. Ferner können sie als Zwischenprodukte zur Herstellung weiterer Arzneimittelwirkstoffe verwendet werden.

Gegenstand der Erfindung sind dementsprechend die Verbindungen der Formel I, ihre Säureadditionssalze sowie ein Verfahren zu ihrer Herstellung, dadurch gekennzeichnet, daß man eine Verbindung der Formel II

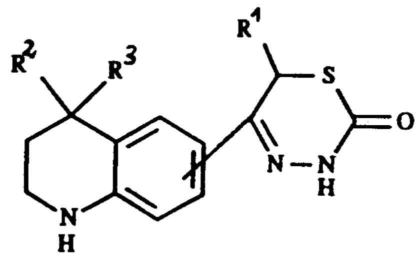

II

worin
$R^1$, $R^2$ und $R^3$ die angegebenen Bedeutungen haben, mit einem Imidchlorid der Formel III

$R^5$-CCl = $NR^4$ III

worin
$R^4$ und $R^5$ die angegebenen Bedeutungen haben, umsetzt oder daß man ein Keton der Formel IV

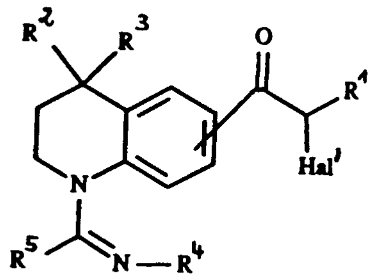

IV

worin
$R^1$, $R^2$, $R^3$, $R^4$ und $R^5$ die angegebenen Bedeutungen haben und Hal' Cl, Br oder I bedeutet, mit einer Verbindung der Formel V

$H_2N$-NH-CS-OR V

worin

R Alkyl mit 1-5 C-Atomen oder ein Äquivalent eines Metall- oder Ammoniumkations bedeutet, umsetzt
oder daß man zur Herstellung einer Verbindung der Formel I, worin $R^4$ = H ist, eine entsprechende Verbindung, die jedoch an Stelle von $R^4$ eine Aminoschutzgruppe trägt, mit einem hydrolysierenden oder hydrogenolysierenden Mittel behandelt oder daß man zur Herstellung einer Verbindung der Formel I, worin $R^4$ = H ist, eine Halogenmagnesiumverbindung der Formel VI

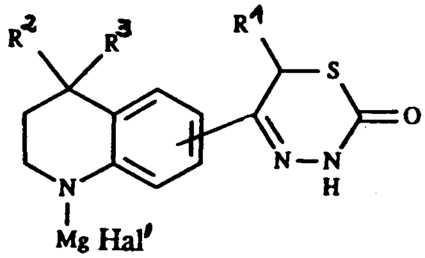

VI

worin
$R^1$, $R^2$, $R^3$ und Hal' die angegebenen Bedeutungen haben, mit einem Nitril der Formel VII

$R^5$-CN VII

worin
$R^5$ die angegebene Bedeutung hat,
umsetzt und das erhaltene Produkt anschließend hydrolysiert
und daß man gegebenenfalls in einem Thiadiazinonderivat der Formel I einen oder beide Rest(e) $R^4$ und/oder $R^5$ in (einen) andere(n) Rest(e) $R^4$ und/oder $R^5$ umwandelt und/oder eine erhaltene Base der Formel I durch Behandeln mit einer Säure in eines ihrer Säureadditionssalze umwandelt.

Vor- und nachstehend haben $R^1$ bis $R^6$, A, Ar, "-alkyl", Hal, Hal' und R die bei den Formeln I, IV bzw. V angegebenen Bedeutungen, wenn nicht ausdrücklich etwas anderes angegeben ist.

In den Formeln steht A für Alkylreste mit 1-8 C-Atomen, die bevorzugt unverzweigt sind, vorzugsweise 1, 2, 3, 4 oder 5 C-Atome besitzen, bevorzugt Methyl, ferner bevorzugt Ethyl oder Propyl, weiterhin bevorzugt Isopropyl, Butyl, Isobutyl, sek-Butyl, tert-Butyl, n-Pentyl oder Isopentyl.

Der Rest Ar kann unsubstituiertes Phenyl sein, ist aber bevorzugt einfach, besonders bevorzugt zweifach substituiertes Phenyl, wobei die Substituenten gleich oder verschieden sein können und vorzugsweise in para-, bzw. in para- und meta-Position stehen. Besonders bevorzugte Substituenten sind Methoxy und Fluor, aber auch Hydroxy, Chlor und Ethoxy.

Im einzelnen ist Ar bevorzugt p-Fluorphenyl oder 3,4-Dimethoxyphenyl.

Die Gruppe "-alkyl" steht für eine geradkettige oder verzweigte Alkylengruppe, vorzugsweise für $-CH_2-$ oder $-CH_2-CH_2-$.

Cycloalkyl kann 3-7 C-Atome enthalten, besitzt aber bevorzugt 5 oder 6 C-Atome und ist vorzugsweise Cyclopentyl oder Cyclohexyl.

Der Dihydrothiadiazinon-Ring steht vorzugsweise in der 6-Stellung, weiterhin bevorzugt in der 7-Stellung des Tetrahydrochinolin-Systems; er kann aber auch in der 5- oder 8-Stellung stehen.

Die Reste $R^1$, $R^2$ und $R^3$ bedeuten vorzugsweise jeweils H oder Methyl.

Der Rest $R^4$ bedeutet vorzugsweise H oder Ethyl, weiterhin bevorzugt Cyclopentyl, Methyl, Propyl, Isopropyl, Butyl oder Isopentyl.

Der Rest $R^5$ bedeutet bevorzugt Phenyl, besonders bevorzugt substituiertes Phenyl, vorzugsweise p-Fluorphenyl oder 3,4-Dimethoxyphenyl.

Gegenstand der Erfindung sind insbesondere diejenigen Verbindungen der Formel I, in denen mindestens einer der genannten Reste eine der vorstehend angegebenen bevorzugten Bedeutungen hat.

Einige bevorzugte Gruppen von Verbindungen können durch die folgenden Teilformeln Ia bis If ausgedrückt werden, die der Formel I entsprechen und worin die nicht näher bezeichneten Reste die bei der Formel I angegebene Bedeutung haben, worin jedoch

in Ia der Dihydrothiadiazinon-Ring in 6-Stellung steht,
- $R^1$, $R^2$ und $R^3$ jeweils H oder Methyl,
- $R^4$ Alkyl mit 1-5 -Atomen und
- $R^5$ ein- oder zweifach substituiertes Phenyl bedeuten;

in Ib der Dihydrothiadiazinon-Ring in 6-Stellung steht,
- $R^1$, $R^2$ und $R^3$ jeweils H oder Methyl,
- $R^4$ Cycloalkyl und
- $R^5$ ein- oder zweifach substituiertes Phenyl bedeuten;

in Ic der Dihydrothiadiazinon-Ring in 6-Stellung steht,
- $R^1$, $R^2$ und $R^3$ jeweils H oder Methyl,
- $R^4$ H und
- $R^5$ ein- oder zweifach substituiertes Phenyl bedeuten;

in Id der Dihydrothiadiazinon-Ring in 6-Stellung steht,
- $R^1$, $R^2$ und $R^3$ jeweils H oder Methyl,
- $R^4$ Alkyl mit 1-5 C-Atomen und
- $R^5$ p-Fluorphenyl, 3-Chlor-4-methoxyphenyl oder 3,4-Dimethoxyphenyl bedeuten;

in Ie der Dihydrothiadiazinon-Ring in 6-Stellung steht,
- $R^1$, $R^2$ und $R^3$ jeweils H oder Methyl,
- $R^4$ Cycloalkyl und
- $R^5$ p-Fluorphenyl, 3-Chlor-4-methoxyphenyl oder 3,4-Dimethoxyphenyl bedeuten;

in If der Dihydrothiadiazinon-Ring in 6-Stellung steht,

| | |
|---|---|
| $R^1$, $R^2$ und $R^3$ | jeweils H oder Methyl, |
| $R^4$ | H und |
| $R^5$ | p-Fluorphenyl, 3-Chlor-4-methoxyphenyl oder 3,4-Dimethoxyhenyl |

bedeuten.

Die Verbindungen der Formel I werden im übrigen nach an sich bekannten Methoden hergestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe für das beanspruchte Verfahren können gewünschtenfalls auch in situ gebildet werden, derart, daß man sie aus dem Reaktionsgemisch nicht isoliert, sondern sofort weiter zu den Verbindungen der Formel I umsetzt.

Die Ausgangsstoffe der Formel II und III sind teilweise bekannt. Sofern sie nicht bekannt sind, können sie nach an sich bekannten Methoden hergestellt werden. Die Herstellung der Verbindungen der Formel II ist aus EP 294 647 bekannt.

Im einzelnen erfolgt die Umsetzung der Verbindungen der Formel II mit den Verbindungen der Formel III in Gegenwart oder Abwesenheit eines inerten Lösungsmittels bei Temperaturen zwischen etwa -20 und etwa +150°, vorzugsweise zwischen 20 und 100°. Als Lösungsmittel eignen sich beispielsweise Kohlenwasserstoffe wie Benzol, Toluol, Xylole oder Mesitylen; halogenierte Kohlenwasserstoffe wie Dichlormethan, Trichlorethylen oder Chlorbenzol; Alkohole wie Methanol, Ethanol oder Isopropanol; Glykole wie Glykolether wie Ethylenglykol, Diethylenglykol, 2-Methoxyethanol; Nitrile wie Acetonitril; Ether wie Tetrahydrofuran oder Dioxan; Amide wie Dimethylformamid (DMF); Sulfoxide wie Dimethylsulfoxid. Auch Gemische dieser Lösungsmittel sind geeignet.

In den Verbindungen der Formel IV steht Hal' bevorzugt für Cl oder Br.

In den Verbindungen der Formel V steht R bevorzugt für Methyl oder Ethyl, aber auch für Na, K oder $NH_4$.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt. Andererseits ist es möglich, die Reaktion stufenweise durchzuführen, wobei man weitere Zwischenprodukte isolieren kann.

Die Ausgangsstoffe der Formeln IV und V sind teilweise bekannt. Sofern sie nicht bekannt sind, können sie nach an sich bekannten Methoden hergestellt werden. Die Ketone der Formel IV sind beispielsweise durch Friedel-Crafts-Synthese aus entsprechenden Tetrahydrochinolinderivaten mit Verbindungen der Formel Hal'-CO-$CHR^2$-Hal' zugänglich.

Im einzelnen erfolgt die Umsetzung der Ketone der Formel IV mit den Verbindungen der Formel V unter Bedingungen, wie sie für die Reaktion zwischen Verbindungen der Formeln II und III zuvor angegeben wurden.

Man kann eine Verbindung der Formel I auch dadurch erhalten, daß man eine Verbindung, die sonst der Formel I entspricht, aber an Stelle von $R^4$ eine "Aminoschutzgruppe" trägt, mit einem Reagenz behandelt, welches diese "Schutzgruppe" reduktiv entfernt.

Als "Schutzgruppen" dienen vorzugsweise $CO_2$-$CH_2C_6H_5$, besonders bevorzugt OH, welches vorzugsweise durch Übergangsmetallcarbonyle, besonders bevorzugt durch Eisenpentacarbonyl, aber auch durch $Fe_2(CO)_9$ bei Temperaturen zwischen etwa -20 und etwa +150°, vorzugsweise zwischen 20 und 100°, in Gegenwart oder Abwesenheit eines inerten Lösungsmittels, entfernt werden kann.

Als Lösungsmittel eignen sich beispielsweise die oben für die Reaktion von II mit III angegebenen.

Verbindungen der Formel I kann man auch dadurch erhalten, daß man eine Halogenmagnesiumverbindung der Formel VI mit einem Nitril der Formel VII umsetzt und das erhaltene Produkt anschließend hydrolysiert.

Verbindungen der Formeln VI und VII sind an sich bekannt oder können nach an sich bekannten Methoden hergestellt werden.

Diese Umsetzungen erfolgen vorzugsweise unter Bedingungen wie sie für Grignard-Reaktionen oder andere organometallischen Reaktionen bekannt sind, zweckmäßig in Gegenwart oder Abwesenheit eines inerten Lösungsmittels bei Temperaturen zwischen etwa -20 und etwa +150°, vorzugsweise zwischen 0 und 150°. Als Lösungsmittel eignen sich die oben angegebenen, soweit sie nicht selbst mit den Verbindungen der Formel VI reagieren können.

Ebenso ist es möglich einen oder beide Rest(e) $R^4$ und/oder $R^5$ in (einen) andere(n) Rest(e) $R^4$ und/oder $R^5$ umzuwandeln. Man kann z.B., unter Verwendung von an sich bekannten Reaktionen, eine $NO_2$-Gruppe zu einer $NH_2$-Gruppe reduzieren, eine $NH_2$- oder NHA-Gruppe alkylieren, eine OH-Gruppe verethern oder aber einen Arylether spalten. Desweiteren können Substituenten dieser Reste $R^4$ und/oder $R^5$, wie z.B. S-$R^6$- oder SO-$R^6$-Gruppen oxidiert werden, sofern die Reaktionen selektiv an den Resten $R^4$ und/oder $R^5$ stattfinden.

Eine Base der Formel I kann mit einer Säure in das zugehörige Säureadditionssalz überführt werden. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasser stoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2- oder 3-Phenylpropionsäure, Citronensäure, Gluconsäure, Ascorbinsäure, Nicotinsäure, Isonicotinsäure, Methan- und Ethansulfonsäure, Ethandisulfonsäure, 2-Hydroxyethansulfonsäure, Benzolsulfonsäure, p-Toluolsulfonsäure, Naphthalin-mono- und disulfonsäuren, Laurylschwefelsäure. Salze mit physiologisch nicht unbedenklichen Säuren, z.B. Pikrate, können zur Aufreinigung der Verbindungen der Formel I verwendet werden.

Verbindungen der Formel I können ein oder mehrere Asymmetriezentren enthalten. In diesem Fall liegen sie gewöhnlich in racemischer Form vor. Erhaltene Racemate können nach an sich bekannten Methoden mechanisch oder chemisch in ihre optischen Antipoden getrennt werden. Vorzugsweise werden aus dem racemischen Gemisch durch Umsetzung mit einem optisch-aktiven Trennmittel Diastereomere gebildet. Als Trennmittel für basische Verbindungen der Formel I eignen sich z.B. optisch aktive Säuren, wie die D- und L-Formen von Weinsäure, Diacetylweinsäure, Dibenzoylweinsäure, Mandelsäure, Äpfelsäure, Milchsäure oder die verschiedenen optisch-aktiven Camphersulfonsäuren, wie $\beta$-Camphersulfonsäure oder aber optisch aktive Camphansäure bzw. andere optisch aktive Terpensäuren.

Natürlich ist es auch möglich, optisch aktive Verbindungen der Formel I nach den oben beschriebenen Methoden zu erhalten, indem man Ausgangsstoffe verwendet, die bereits optisch aktiv sind.

Darüber hinaus zeigen die Verbindungen der Formel I, in Analogie zur E-Z-Isomerie bei C=C-Doppelbindungen, eine vergleichbare Stereoisomerie an der nicht-cyclischen C=N-Doppelbindung. Die Verbindungen können somit als Mischungen der möglichen Stereoisomeren oder aber als reine E- oder Z-Isomere vorliegen.

Gegenstand der Erfindung ist ferner die Verwendung der Verbindungen der Formel I und ihrer physiologischen unbedenklichen Salze zur Herstellung pharmazeutischer Zubereitungen, insbesondere auf nicht-chemische Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind ferner Mittel, insbesondere pharmazeutische Zubereitungen, enthaltend mindestens eine Verbindung der Formel I und/oder eines ihrer physiologisch unbedenklichen Salze.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielweise Wasser, pflanzliche Öle, Benzylalkohole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Dragees, Kapseln, Sirupe, Säfte oder Tropfen, zur rektalen Anwendung Suppositorien, zur parenteralen Anwendung Lösungen, vorzugsweise ölige oder wässerige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z.B. ein oder mehrere Vitamine.

Die Verbindungen der Formel I können bei der Bekämpfung von Krankheiten, insbesondere von Herzinsuffizienz sowie bei der therapeutischen Behandlung des menschlichen oder tierischen Körpers verwendet werden.

Dabei werden die erfindungsgemäßen Substanzen in der Regel in Analogie zu bekannten positiv inotrop wirksamen Substanzen wie Amrinon verabreicht, vorzugsweise in Dosierungen zwischen etwa 1 und 100 mg, insbesondere zwischen 2 und 20 mg pro Dosierungseinheit. Die tägliche Dosierung liegt vorzugsweise zwischen etwa 0,02 und 2 mg/kg Körpergewicht. Die spezielle Dosis für jeden bestimmten Patienten hängt jedoch von den verschiedensten Faktoren ab, beispielsweise von der Wirksamkeit der eingesetzten

speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt. Im Vergleich zu den bisher zur Therapie der Herzinsuffizienz verwendeten Digitalis-Glykosiden zeichnen sich die Verbindungen der Formel I durch verbesserte therapeutische Breite und periphere Entlastung aus.

In den nachfolgenden Beispielen bedeutet "übliche Aufarbeitung":
Man gibt, falls erforderlich, Wasser oder verdünnte Natronlauge hinzu, extrahiert mit einem organischen Lösungsmittel wie Ethylacetat, Chloroform oder Dichlormethan, trennt ab, trocknet die organische Phase über Natriumsulfat, filtriert, dampft ein und reinigt durch Chromatographie und/oder Kristallisation.

Vor- und nachstehend sind alle Temperaturen in Celsiusgraden angegeben. Die angegebenen Festpunkte beziehen sich auf die freien Basen und/oder die zugrundeliegenden Hydrochloride.

## Beispiel 1

Eine Lösung von 2,6 g 5-(1,2,3,4-Tetrahydrochinolin-6-yl)-6-methyl-3,6-dihydro-2H-1,3,4-thiadiazin-2-on ("A") in 40 ml Dichlormethan, versetzt mit 1 ml Pyridin, wird mit 2,1 g N-Ethyl-3,4-dimethoxybenzoesäureimidchlorid, gelöst in 20 ml Dichlormethan, in der Kälte versetzt und 1 Stunde gerührt. Nach Entfernung des Lösungsmittels arbeitet man wie üblich auf und erhält 5-[1-(N-Ethyl-3,4-dimethoxybenzimidoyl)-1,2,3,4-tetrahydrochinolin-6-yl]-6-methyl-3,6-dihydro-2H-1,3,4-thiadiazin-2-on, F. 208°. Hydrochlorid, F. 247°.

Analog erhält man:
aus "A" und N-Isopropyl-3,4-dimethoxybenzoesäureimidchlorid
5-[1-(N-Isopropyl-3,4-dimethoxybenzimidoyl)-1,2,3,4-tetrahydrochinolin-6-yl]-6-methyl-3,6-dihydro-2H-1,3,4-thiadiazin-2-on, F. 250°;
aus "A" und N-Cyclopentyl-3,4-dimethoxybenzoesäureimidchlorid
5-[1-(N-Cyclopentyl-3,4-dimethoxybenzimidoyl)-1,2,3,4-tetrahydrochinolin-6-yl]-6-methyl-3,6-dihydro-2H-1,3,4-thiadiazin-2-on, F. 258°;
aus "A" und N-(2-Methylbutyl)-3,4-dimethoxybenzoesäureimidchlorid
5-[1-(N-(2-Methylbutyl)-3,4-dimethoxybenzimidoyl)-1,2,3,4-tetrahydrochinolin-6-yl]-6-methyl-3,6-dihydro-2H-1,3,4-thiadiazin-2-on, F. 253°;
aus "A" und N-Cyclopropyl-3,4-dimethoxybenzoesäureimidchlorid
5-[1-(N-Cyclopropyl-3,4-dimethoxybenzimidoyl)-1,2,3,4-tetrahydrochinolin-6-yl]-6-methyl-3,6-dihydro-2H-1,3,4-thiadiazin-2-on, F. 218°; Hydrochlorid F. 242°;
aus "A" und N-Methyl-3-chlor-4-methoxybenzoesäureimidchlorid
5-[1-(N-Methyl-3-chlor-4-methoxybenzimidoyl)-1,2,3,4-tetrahydrochinolin-6-yl]-6-methyl-3,6-dihydro-2H-1,3,4-thiadiazin-2-on, F. 229°; Hydrochlorid F. 252°;
aus "A" und N-Isopropyl-3-chlor-4-methoxybenzoesäureimidchlorid
5-[1-(N-Isopropyl-3-chlor-4-methoxybenzimidoyl)-1,2,3,4-tetrahydrochinolin-6-yl]-6-methyl-3,6-dihydro-2H-1,3,4-thiadiazin-2-on, F. 240°;
aus "A" und N-Phenyl-3,4-dimethoxybenzoesäureimidchlorid
5-[1-(N-Phenyl-3,4-dimethoxybenzimidoyl)-1,2,3,4-tetrahydrochinolin-6-yl]-6-methyl-3,6-dihydro-2H-1,3,4-thiadiazin-2-on;
aus "A" und N-Phenyl-3-chlor-4-methoxybenzoesäureimidchlorid
5-[1-(N-Phenyl-3-chlor-4-methoxybenzimidoyl)-1,2,3,4-tetrahydrochinolin-6-yl]-6-methyl-3,6-dihydro-2H-1,3,4-thiadiazin-2-on;
aus "A" und N-Ethyl-p-fluorbenzoesäureimidchlorid
5-[1-(N-Ethyl-p-fluorbenzimidoyl)-1,2,3,4-tetrahydrochinolin-6-yl]-6-methyl-3,6-dihydro-2H-1,3,4-thiadiazin-2-on, F. 264°;
aus "A" und N-Butyl-3,4-dimethoxybenzoesäureimidchlorid
5-[1-(N-Butyl-3,4-dimethoxybenzimidoyl)-1,2,3,4-tetrahydrochinolin-6-yl]-6-methyl-3,6-dihydro-2H-1,3,4-thiadiazin-2-on.

## Beispiel 2

Analog Beispiel 1 erhält man aus 5-(4,4-Dimethyl-1,2,3,4-tetrahydrochinolin-6-yl)-6-methyl-3,6-dihydro-2H-1,3,4-thiadiazin-2-on ("B") und N-Butyl-3,4-dimethoxybenzoesäureimidchlorid 5-[1-(N-Butyl-3,4-dimethoxybenzimidoyl)-4,4-dimethyl-1,2,3,4-tetrahydrochinolin-6-yl]-6-methyl-3,6-dihydro-2H-1,3,4-thiadiazin-2-on, F. 253°.

Analog erhält man:

aus "B" und N-Cyclohexyl-3,4-dimethoxybenzoesäureimidchlorid
5-[1-(N-Cyclohexyl-3,4-dimethoxybenzimidoyl)-4,4-dimethyl-1,2,3,4-tetrahydrochinolin-6-yl]-6-methyl-3,6-dihydro-2H-1,3,4-thiadiazin-2-on;
aus "B" und N-Cyclopropyl-3,4-dimethoxybenzoesäureimidchlorid
5-[1-(N-Cyclopropyl-3,4-dimethoxybenzimidoyl)-4,4-dimethyl-1,2,3,4-tetrahydrochinolin-6-yl]-6-methyl-3,6-dihydro-2H-1,3,4-thiadiazin-2-on;
aus "B" und N-Cyclopentyl-3,4-dimethoxybenzoesäureimidchlorid
5-[1-(N-Cyclopentyl-3,4-dimethoxybenzimidoyl)-4,4-dimethyl-1,2,3,4-tetrahydrochinolin-6-yl]-6-methyl-3,6-dihydro-2H-1,3,4-thiadiazin-2-on;
aus "B" und N-Ethyl-3,4-dimethoxybenzoesäureimidchlorid
5-[1-(N-Ethyl-3,4-dimethoxybenzimidoyl)-4,4-dimethyl-1,2,3,4-tetrahydrochinolin-6-yl]-6-methyl-3,6-dihydro-2H-1,3,4-thiadiazin-2-on;
aus "B" und N-Ethyl-p-fluorbenzoesäureimidchlorid
5-[1-(N-Ethyl-p-fluorbenzimidoyl)-4,4-dimethyl-1,2,3,4-tetrahydrochinolin-6-yl]-6-methyl-3,6-dihydro-2H-1,3,4-thiadiazin-2-on;
aus "B" und N-Isopropyl-p-fluorbenzoesäureimidchlorid
5-[1-(N-Isopropyl-p-fluorbenzimidoyl)-4,4-dimethyl-1,2,3,4-tetrahydrochinolin-6-yl]-6-methyl-3,6-dihydro-2H-1,3,4-thiadiazin-2-on;
aus "B" und N-Methyl-3-chlor-4-methoxybenzoesäureimidchlorid
5-[1-(N-Methyl-3-chlor-4-methoxybenzimidoyl)-4,4-dimethyl-1,2,3,4-tetrahydrochinolin-6-yl]-6-methyl-3,6-dihydro-2H-1,3,4-thiadiazin2-on;
aus "B" und N-Isopropyl-3-chlor-4-methoxybenzoesäureimidchlorid
5-[1-(N-Isopropyl-3-chlor-4-methoxybenzimidoyl)-4,4-dimethyl-1,2,3,4-tetrahydrochinolin-6-yl]-6-methyl-3,6-dihydro-2H-1,3,4-thiadiazin-2-on.

## Beispiel 3

Zu einer Lösung von 2,9 g 5-[1-(N-Hydroxy-3,4-dimethoxybenzimidoyl)-1,2,3,4-tetrahydrochinolin-6-yl]-6-methyl-3,6-dihydro-2H-1,3,4-thiadiazin-2-on (erhältlich aus N-Hydroxy-3,4-dimethoxybenzoesäureimidchlorid) und 5-(1,2,3,4-Tetrahydrochinolin-6-yl)-6-methyl-3,6-dihydro-2H-1,3,4-thiadiazin-2-on) in 30 ml Tetrahydrofuran wird 1 Äquivalent Eisenpentacarbonyl zugetropft. Die Reaktionsmischung wird 2 Stunden gekocht und wie üblich aufgearbeitet. Man erhält 5-(1-(3,4-Dimethoxybenzimidoyl)-1,2,3,4-tetrahydrochinolin-6-yl)]-6-methyl-3,6-dihydro-2H-1,3,4-thiadiazin-2-on, F. 228°.

Analog erhält man durch Kochen mit Eisenpentacarbonyl:
aus 5-[1-(N-Hydroxy-p-fluorbenzimidoyl)-1,2,3,4-tetrahydrochinolin-6-yl]-6-methyl-3,6-dihydro-2H-1,3,4-thiadiazin-2-on das 5-[1-(p-Fluorbenzimidoyl)-1,2,3,4-tetrahydrochinolin-6-yl]-6-methyl-3,6-dihydro-2H-1,3,4-thiadiazin-2-on, F. 267°;
aus 5-[1-(N-Hydroxy-3,4-dimethoxybenzimidoyl)-4,4-dimethyl-1,2,3,4-tetrahydrochinolin-6-yl]-6-methyl-3,6-dihydro-2H-1,3,4-thiadiazin-2-on das 5-[1-(3,4-Dimethoxybenzimidoyl)-4,4-dimethyl-1,2,3,4-tetrahydrochinolin-6-yl]-6-methyl-3,6-dihydro-2H-1,3,4-thiadiazin-2-on;
aus 5-[1-(N-Hydroxy-p-fluorbenzimidoyl)-4,4-dimethyl-1,2,3,4-tetrahydrochinolin-6-yl]-6-methyl-3,6-dihydro-2H-1,3,4-thiadiazin-2-on das 5-[1-(p-Fluorbenzimidoyl)-4,4-dimethyl-1,2,3,4-tetrahydrochinolin-6-yl]-6-methyl-3,6-dihydro-2H-1,3,4-thiadiazin-2-on.
aus 5-[1-(N-Hydroxy-3-chlor-4-methoxybenzimidoyl)-1,2,3,4-tetrahydrochinolin-6-yl]-6-methyl-3,6-dihydro-2H-1,3,4-thiadiazin-2-on das 5-[1-(3-Chlor-4-methoxybenzimidoyl)-1,2,3,4-tetrahydrochinolin-6-yl]-6-methyl-3,6-dihydro-2H-1,3,4-thiadiazin-2-on;
aus 5-[1-(N-Hydroxy-p-fluorbenzimidoyl)-1,2,3,4-tetrahydrochinolin-6-yl]-6-ethyl-3,6-dihydro-2H-1,3,4-thiadiazin-2-on das 5-[1-(p-Fluorbenzimidoyl)-1,2,3,4-tetrahydrochinolin-6-yl]-6-ethyl-3,6-dihydro-2H-1,3,4-thiadiazin-2-on;
aus 5-[1-(N-Hydroxy-3-chlor-4-methoxybenzimidoyl)-4,4-dimethyl-1,2,3,4-tetrahydrochinolin-6-yl]-6-ethyl-3,6-dihydro-2H-1,3,4-thiadiazin-2-on das 5-[1-(3-Chlor-4-methoxybenzimidoyl)-4,4-dimethyl-1,2,3,4-tetrahydrochinolin-6-yl]-6-ethyl-3,6-dihydro-2H-1,3,4-thiadiazin-2-on;
aus 5-[1-(N-Hydroxy-p-fluorbenzimidoyl)-4,4-dimethyl-1,2,3,4-tetrahydrochinolin-6-yl]-6-isopropyl-3,6-dihydro-2H-1,3,4-thiadiazin-2-on das 5-[1-(p-Fluorbenzimidoyl)-4,4-dimethyl-1,2,3,4-tetrahydrochinolin-6-yl]-6-isopropyl-3,6-dihydro-2H-1,3,4-thiadiazin-2-on;
aus 5-[1(N-Hydroxy-benzimidoyl)-1,2,3,4-tetrahydrochinolin-6-yl]-6-methyl-3,6-dihydro-2H-1,3,4-thiadiazin-2-on das 5-[1-Benzimidoyl-1,2,3,4-tetrahydrochinolin-6-yl]-6-methyl-3,6-dihydro-2H-1,3,4-thiadiazin-2-on;
aus 5-[1-(N-Hydroxy-3,4-dimethoxybenzimidoyl)-4,4-dimethyl-1,2,3,4-tetrahydrochinolin-6-yl]-6-ethyl-3,6-dihydro-2H-1,3,4-thiadiazin-2-on das 5-[1-(3,4-Dimethoxybenzimidoyl)-4,4-dimethyl-1,2,3,4-tetrahydrochinolin-6-yl]-6-ethyl-3,6-dihydro-2H-1,3,4-thiadiazin-2-on;
aus 5-[1-(N-Hydroxy-p-fluorbenzimidoyl)-1,2,3,4-tetrahydrochinolin-6-yl]-6-ethyl-3,6-dihydro-2H-1,3,4-thiadiazin-2-on das 5-[1-(p-Fluorbenzimidoyl)-1,2,3,4-tetrahydrochinolin-6-yl]-6-ethyl-3,6-dihydro-2H-1,3,4-thiadiazin-2-on.

Beispiel 4

Eine Lösung von 3,1 g 6-(6-Methyl-2-oxo-3,6-dihydro-2H-1,3,4-thiadiazin-5-yl)-1,2,3,4-tetrahydrochinolin-1-magnesiumbromid ("C") in 30 ml Tetrahydrofuran wird tropfenweise mit 1 Äquivalent p-Fluorbenzonitril versetzt und 1 Stunde gekocht. Man erhält nach üblicher Aufarbeitung 5-[1-(p-Fluorbenzimidoyl)-1,2,3,4-tetrahydrochinolin-6-yl]-6-methyl-3,6-dihydro-2H-1,3,4-thiadiazin-2-on.

Analog erhält man aus "C"
mit Benzonitril das 5-(1-Benzimidoyl-1,2,3,4-tetrahydrochinolin-6-yl)-6-methyl-3,6-dihydro-2H-1,3,4-thiadiazin-2-on;
mit p-Chlorbenzonitril das 5-[1-(p-Chlorbenzimidoyl-1,2,3,4-tetrahydrochinolin-6-yl]-6-methyl-3,6-dihydro-2H-1,3,4-thiadiazin-2-on.

Beispiel 5

Eine Lösung von 2,5 g 1-(N-Isopropyl-p-fluorbenzimidoyl)-6-(2-chlorpropionyl)-1,2,3,4-tetrahydrochinolin in 40 ml Acetonitril wird mit 1 Äquivalent Hydrazinthioameisensäure-0-ethylester versetzt und 2 Std. gekocht.

Man erhält nach üblicher Aufarbeitung
5-[1-(N-Isopropyl-p-fluorbenzimidoyl)-1,2,3,4-tetrahydrochinolin-6-yl]-6-methyl-3,6-dihydro-2H-1,3,4-thiadiazin-2-on.

Beispiel 6

Analog Beispiel 1 erhält man aus 5-(4,4-Dimethyl-1,2,3,4-tetrahydrochinolin-6-yl)-6-ethyl-3,6-dihydro-2H-1,3,4-thiadiazin-2-on ("D") und N-Butyl-3,4-dimethoxybenzoesäureimidchlorid nach üblicher Aufarbeitung, 5-[1-(N-Butyl-3,4-dimethoxybenzimidoyl)-4,4-dimethyl-1,2,3,4-tetrahydrochinolin-6-yl]-6-ethyl-3,6-dihydro-2H-1,3,4-thiadiazin-2-on.

Analog erhält man:
aus "D" und N-Cyclohexyl-3,4-dimethoxybenzoesäureimidchlorid
5-[1-(N-Cyclohexyl-3,4-dimethoxybenzimidoyl)-4,4-dimethyl-1,2,3,4-tetrahydrochinolin-6-yl]-6-ethyl-3,6-dihydro-2H-1,3,4-thiadiazin-2-on;
aus "D" und N-Cyclopropyl-3,4-dimethoxybenzoesäureimidchlorid
5-[1-(N-Cyclopropyl-3,4-dimethoxybenzimidoyl)-4,4-dimethyl-1,2,3,4-tetrahydrochinolin-6-yl]-6-ethyl-3,6-dihydro-2H-1,3,4-thiadiazin-2-on;
aus "D" und N-Cyclopentyl-3,4-dimethoxybenzoesäureimidchlorid
5-[1-(N-Cyclopentyl-3,4-dimethoxybenzimidoyl)-4,4-dimethyl-1,2,3,4-tetrahydrochinolin-6-yl]-6-ethyl-3,6-dihydro-2H-1,3,4-thiadiazin-2-on;
aus "D" und N-Ethyl-3,4-dimethoxybenzoesäureimidchlorid
5-[1-(N-Ethyl-3,4-dimethoxybenzimidoyl)-4,4-dimethyl-1,2,3,4-tetrahydrochinolin-6-yl]-6-ethyl-3,6-dihydro-2H-1,3,4-thiadiazin-2-on;
aus "D" und N-Ethyl-p-fluorbenzoesäureimidchlorid
5-[1-(N-Ethyl-p-fluorbenzimidoyl)-4,4-dimethyl-1,2,3,4-tetrahydrochinolin-6-yl]-6-ethyl-3,6-dihydro-2H-1,3,4-thiadiazin-2-on;
aus "D" und N-Isopropyl-p-fluorbenzoesäureimidchlorid
5-[1-(N-Isopropyl-p-fluorbenzimidoyl)-4,4-dimethyl-1,2,3,4-tetrahydrochinolin-6-yl]6-ethyl-3,6-dihydro-2H-1,3,4-thiadiazin-2-on;
aus "D" und N-Methyl-3-chlor-4-methoxybenzoesäureimidchlorid
5-[1-(N-Methyl-3-chlor-4-methoxybenzimidoyl)-4,4-dimethyl-1,2,3,4-tetrahydrochinolin-6-yl]-6-ethyl-3,6-dihydro-2H-1,3,4-thiadiazin-2-on;
aus "D" und N-Isopropyl-3-chlor-4-methoxybenzoesäureimidchlorid
5-[1-(N-Isopropyl-3-chlor-4-methoxybenzimidoyl)-4,4-dimethyl-1,2,3,4-tetrahydrochinolin-6-yl]-6-ethyl-3,6-dihydro-2H-1,3,4-thiadiazin-2-on.

Beispiel 7

Analog Beispiel 1 erhält man aus 5-(1,2,3,4-Tetrahydrochinolin-6-yl)-6-ethyl-3,6-dihydro-2H-1,3,4-thiadiazin-2-on ("E") und N-Butyl-3,4-dimethoxybenzoesäureimidchlorid, nach üblicher Aufarbeitung, 5-[1-(N-Butyl-3,4-dimethoxybenzimidoyl)-4,4-dimethyl-1,2,3,4-tetrahydrochinolin-6-yl]-6-ethyl-3,6-dihydro-2H-1,3,4-thiadiazin-2-on.

Analog erhält man:

aus "E" und N-Cyclohexyl-3,4-dimethoxybenzoesäureimidchlorid
5-[1-(N-Cyclohexyl-3,4-dimethoxybenzimidoyl)-1,2,3,4-tetrahydrochinolin-6-yl]-6-ethyl-3,6-dihydro-2H-1,3,4-thiadiazin-2-on;
aus "E" und N-Cyclopropyl-3,4-dimethoxybenzoesäureimidchlorid
5-[1-(N-Cyclopropyl-3,4-dimethoxybenzimidoyl)-1,2,3,4-tetrahydrochinolin-6-yl]-6-ethyl-3,6-dihydro-2H-1,3,4-thiadiazin-2-on;
aus "E" und N-Phenyl-3,4-dimethoxybenzoesäureimidchlorid
5-[1-(N-Phenyl-3,4-dimethoxybenzimidoyl)-1,2,3,4-tetrahydrochinolin-6-yl]-6-ethyl-3,6-dihydro-2H-1,3,4-thiadiazin-2-on;
aus "E" und N-Cyclopentyl-3,4-dimethoxybenzoesäureimidchlorid
5-[1-(N-Cyclopentyl-3,4-dimethoxybenzimidoyl)-1,2,3,4-tetrahydrochinolin-6-yl]-6-ethyl-3,6-dihydro-2H-1,3,4-thiadiazin-2-on;
aus "E" und N-Ethyl-3,4-dimethoxybenzoesäureimidchlorid
5-[1-(N-Ethyl-3,4-dimethoxybenzimidoyl)-1,2,3,4-tetrahydrochinolin-6-yl]-6-ethyl-3,6-dihydro-2H-1,3,4-thiadiazin-2-on, F. 171°;
aus "E" und N-Ethyl-p-fluorbenzoesäureimidchlorid
5-[1-(N-Ethyl-p-fluorbenzimidoyl)-1,2,3,4-tetrahydrochinolin-6-yl]-6-ethyl-3,6-dihydro-2H-1,3,4-thiadiazin-2-on;
aus "E" und N-Isopropyl-p-fluorbenzoesäureimidchlorid
5-[1-(N-Isopropyl-p-fluorbenzimidoyl)-1,2,3,4-tetrahydrochinolin-6-yl]-6-ethyl-3,6-dihydro-2H-1,3,4-thiadiazin-2-on;
aus "E" und N-Methyl-3-chlor-4-methoxybenzoesäureimidchlorid
5-[1-(N-Methyl-3-chlor-4-methoxybenzimidoyl)-1,2,3,4-tetrahydrochinolin-6-yl]-6-ethyl-3,6-dihydro-2H-1,3,4-thiadiazin-2-on;
aus "E" und N-Isopropyl-3-chlor-4-methoxybenzoesäureimidchlorid
5-[1-(N-Isopropyl-3-chlor-4-methoxybenzimidoyl)-1,2,3,4-tetrahydrochinolin-6-yl]-6-ethyl-3,6-dihydro-2H-1,3,4-thiadiazin-2-on.

Beispiel 8

Eine Lösung von 7,2 g 5-[1-(N-Ethyl-3,4-dimethoxybenzimidoyl)-1,2,3,4-tetrahydrochinolin-6-yl]-6-methyl-3,6-dihydro-2H-1,3,4-thiadiazin-2-on ("F") in 140 ml Tetrahydrofuran wird mit einer entsprechenden Menge von S-(+)-Campher-10-sulfonsäure versetzt, so daß die entsprechenden Diastereomeren in kristalliner Form enthalten werden. Die Durchführung der Racematspaltung und die Isolierung der reinen Enantiomeren erfolgt nach der üblichen, an sich bekannten Verfahrensweise.

Man erhält die beiden optisch Antipoden:

(+) - "F" F. 173°, Hydrochlorid F. 252°; $[\alpha]_D^{20} = +439{,}3°$;
(-) - "F" F. 173°, Hydrochlorid F. 252°, $[\alpha]_D^{20} = -433{,}9°$;
(in beiden Fällen: c = 1 in Methanol).

Beispiel 9

Analog Beispiel 1 erhält man aus 5-(1,2,3,4-Tetrahydrochinolin-6-yl)-3,6-dihydro-2H-1,3,4-thiadiazin-2-on ("G") und N-Butyl-3,4-dimethoxybenzoesäureimidchlorid 5-[1-(N-Butyl-3,4-dimethoxybenzimidoyl)-1,2,3,4-tetrahydrochinolin-6-yl]-3,6-dihydro-2H-1,3,4-thiadiazin-2-on.

Analog erhält man:

aus "G" und N-Isopropyl-3,4-dimethoxybenzoesäureimidchlorid
5-[1-(N-Isopropyl-3,4-dimethoxybenzimidoyl)-1,2,3,4-tetrahydrochinolin-6-yl]-3,6-dihydro-2H-1,3,4-thiadiazin-2-on;
aus "G" und N-Cyclopentyl-3,4-dimethoxybenzoesäureimidchlorid
5-[1-(N-Cyclopentyl-3,4-dimethoxybenzimidoyl)-1,2,3,4-tetrahydrochinolin-6-yl]-3,6-dihydro-2H-1,3,4-thiadiazin-2-on;

aus "G" und N-(2-Methylbutyl)-3,4-dimethoxybenzoesäureimidchlorid
5-[1-(N-(2-Methylbutyl)-3,4-dimethoxybenzimidoyl)-1,2,3,4-tetrahydrochinolin-6-yl]-3,6-dihydro-2H-1,3,4-thiadiazin-2-on;
aus "G" und N-Ethyl-3,4-dimethoxybenzoesäureimidchlorid
5-[1-(N-Ethyl-3,4-dimethoxybenzimidoyl)-1,2,3,4-tetrahydrochinolin-6-yl]-3,6-dihydro-2H-1,3,4-thiadiazin-2-on;
aus "G" und N-Cyclopropyl-3,4-dimethoxybenzoesäureimidchlorid
5-[1-(N-Cyclopropyl-3,4-dimethoxybenzimidoyl)-1,2,3,4-tetrahydrochinolin-6-yl]-3,6-dihydro-2H-1,3,4-thiadiazin-2-on;
aus "G" und N-Methyl-3-chlor-4-methoxybenzoesäureimidchlorid
5-[1-(N-Methyl-3-chlor-4-methoxybenzimidoyl)-1,2,3,4-tetrahydrochinolin-6-yl]-3,6-dihydro-2H-1,3,4-thiadiazin-2-on, Hydrochlorid F. 252°;
aus "G" und N-Isopropyl-3-chlor-4-methoxybenzoesäureimidchlorid
5-[1-(N-Isopropyl-3-chlor-4-methoxybenzimidoyl)-1,2,3,4-tetrahydrochinolin-6-yl]-3,6-dihydro-2H-1,3,4-thiadiazin-2-on;
aus "G" und N-Phenyl-3,4-dimethoxybenzoesäureimidchlorid
5-[1-(N-Phenyl-3,4-dimethoxybenzimidoyl)-1,2,3,4-tetrahydrochinolin-6-yl]-3,6-dihydro-2H-1,3,4-thiadiazin-2-on;
aus "G" und N-Phenyl-3-chlor-4-methoxybenzoesäureimidchlorid
5-[1-(N-Phenyl-3-chlor-4-methoxybenzimidoyl)-1,2,3,4-tetrahydrochinolin-6-yl]-3,6-dihydro-2H-1,3,4-thiadiazin-2-on.

Die nachstehenden Beispiele betreffen pharmazeutische Zubereitungen, die Verbindungen der Formel I oder ihre Säureadditionssalze enthalten.

Beispiel A: Tabletten

Ein Gemisch von 1 kg 5-[1-(N-Ethyl-3,4-dimethoxybenzimidoyl)-1,2,3,4-tetrahydrochinolin-6-yl]-6-methyl-3,6-dihydro-2H-1,3,4-thiadiazin-2-on, 10 kg Lactose, 6 kg mikrokristalline Cellulose, 6 kg Kartoffelstärke, 1 kg Polyvinylpyrrolidon, 0,8 g Talk und 0,1 kg Magnesiumstearat wird in üblicher Weise zu Tabletten gepreßt, derart, daß jede Tablette 10 mg Wirkstoff enthält.

Beispiel B: Dragees

Analog Beispiel A werden Tabletten gepreßt, die anschließend in üblicher weise mit einem Überzug aus Saccharose, Kartoffelstärke, Talk, Tragant und Farbstoff überzogen werden.

Beispiel C: Kapseln

1 kg 5-[1-(N-Butyl-3,4-dimethoxybenzimidoyl)-4,4-dimethyl-1,2,3,4-tetrahydrochinolin-6-yl)-6-methyl-3,6-dihydro-2H-1,3,4-thiadiazin-2-on wird in üblicher Weise in Hartgelatinekapseln gefüllt, so daß jede Kapsel 5 mg Wirkstoff enthält.

Beispiel D: Ampullen

Eine Lösung von 1 kg 5[1-(N-Ethyl-p-fluorbenzimidoyl)-1,2,3,4-tetrahydrochinolin-6-yl]-6-methyl-3,6-dihydro-2H-1,3,4-thiadiazin-2-on in 30 l 1,2-Propandiol wird steril filtriert, in Ampullen abgefüllt, unter sterilen Bedingungen lyophilisiert und steril verschlossen. Jede Ampulle enthält 2 mg Wirkstoff.

Analog sind Tabletten, Dragees, Kapseln und Ampullen erhältlich, die einen der übrigen Wirkstoffe der Formel I und/oder ihre physiologisch unbedenklichen Säureadditionssalze enthalten.

## Patentansprüche

**1.** Thiadiazinonderivate der Formel I,

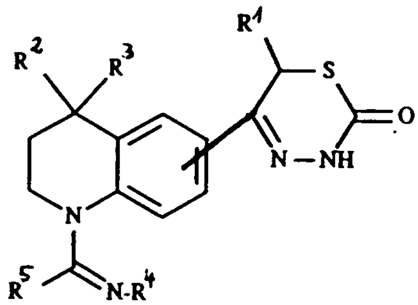

I

worin

| | |
|---|---|
| $R^1$, $R^2$ und $R^3$ | jeweils H oder A, |
| $R^4$ | H, A, Ar, Ar-alkyl oder Cycloalkyl mit 3-7 C-Atomen, |
| $R^5$ | A, Ar oder Cycloalkyl mit 3-7 C-Atomen, |
| A | Alkyl mit 1-8 C-Atomen, |
| Ar | einen unsubstituierten oder einen ein-, zwei- oder dreifach durch A, OH, OA, $SR^6$, $SOR^6$, $SO_2R^6$, $NO_2$, CO-A, O-$CH_2$-O, Hal, $NH_2$, NHA oder $NA_2$ substituierten Phenylrest, |
| "-alkyl" | Alkylen mit 1-5 C-Atomen, |
| $R^6$ | A oder Ar und |
| Hal | F, Cl, Br oder I bedeuten |

sowie deren Salze.

**2.**

a) 5-[1-(N-Isopropyl-3,4-dimethoxybenzimidoyl)-1,2,3,4-tetrahydrochinolin-6-yl]-6-methyl-3,6-dihydro-2H-1,3,4-thiadiazin-2-on;

b) 5-[1-(N-Cyclopentyl-3,4-dimethoxybenzimidoyl)-1,2,3,4-tetrahydrochinolin-6-yl]-6-methyl-3,6-dihydro-2H-1,3,4-thiadiazin-2-on;

c) 5-[1-(N-(2-Methylbutyl)-3,4-dimethoxybenzimidoyl)-1,2,3,4-tetrahydrochinolin-6-yl]-6-methyl-3,6-dihydro-2H-1,3,4-thiadiazin-2-on;

d) 5-[1-(N-Ethyl-3,4-dimethoxybenzimidoyl)-1,2,3,4-tetrahydrochinolin-6-yl]-6-methyl-3,6-dihydro-2H-1,3,4-thiadiazin-2-on;

e) 5-[1-(N-1-Butyl-3,4-dimethoxybenzimidoyl)-1,2,3,4-tetrahydrochinolin-6-yl]-6-methyl-3,6-dihydro-2H-1,3,4-thiadiazin-2-on;

f) 5-[1-(N-Ethyl-4-fluorbenzimidoyl)-1,2,3,4-tetrahydrochinolin-6-yl]-6-methyl-3,6-dihydro-2H-1,3,4-thiadiazin-2-on.

g) 5-[1-(3,4-Dimethoxybenzimidoyl)-1,2,3,4-tetrahydrochinolin-6-yl]-6-methyl-3,4-dihydro-2H-1,3,4-thiadiazin-2-on.

**3.** Verfahren zur Herstellung von Thiadiazinonen der Formel I nach Anspruch 1 sowie von deren Salzen, dadurch gekennzeichnet, daß man eine Verbindung der Formel II

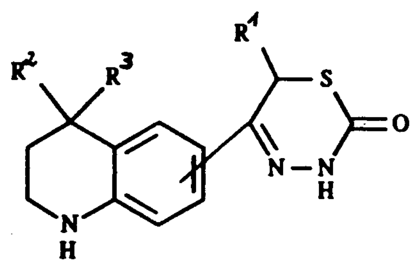

II

$R^1$, $R^2$ und $R^3$ die angegebenen Bedeutungen haben, mit einem Imidchlorid der Formel III

$R^5$-CCl = $NR^4$ III

worin

$R^4$ und $R^5$ die angegebenen Bedeutungen haben, umsetzt

oder daß man ein Keton der Formel IV

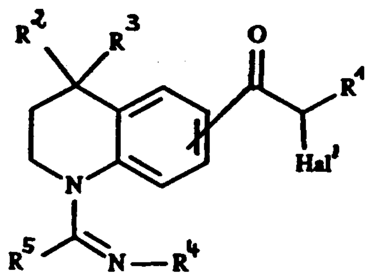

IV

worin

$R^1$, $R^2$, $R^3$, $R^4$ und $R^5$ die in Anspruch 1 angegebenen Bedeutungen haben und

Hal', Cl, Br oder I bedeutet,

mit einer Verbindung der Formel V

$H_2N$-NH-CS-OR V

worin

R Alkyl mit 1-5 C-Atomen oder ein Äquivalent eines Metall- oder Ammoniumkations bedeutet, umsetzt

oder daß man zur Herstellung einer Verbindung der Formel I, worin $R^4$ = H ist, eine entsprechende Verbindung, die jedoch an Stelle von $R^4$ eine Aminoschutzgruppe trägt, mit einem hydrolysierenden oder hydrogenolysierenden Mittel behandelt

oder daß man zur Herstellung einer Verbindung der Formel I, worin $R^4$ = H ist, eine Halogenmagnesiumverbindung der Formel VI

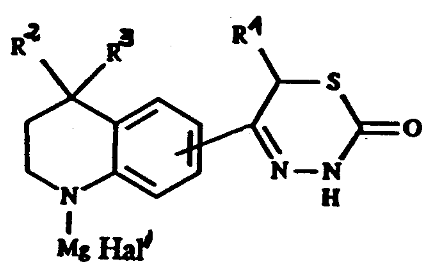

VI

worin

$R^1$, $R^2$, $R^3$ und Hal' die angegebenen Bedeutungen haben,

mit einem Nitril der Formel VII

$R^5$-CN VII

worin

$R^5$ die angegebene Bedeutung hat,

umsetzt und daß erhaltene Produkt anschließend hydrolisiert

und daß man gegebenenfalls in einem Thiadiazinonderivat der Formel I einen oder beide Rest(e) $R^4$ und/oder $R^5$ in (einen) andere(n) Rest(e) $R^4$ und/oder $R^5$ umwandelt und/oder eine erhaltene Base der Formel I durch Behandeln mit einer Säure in eines ihrer Säureadditionssalze umwandelt.

**4.** Verfahren zur Herstellung pharmazeutischer Zubereitungen, dadurch gekennzeichnet, daß man eine Verbindung der Formel I und/oder eines ihrer physiologisch unbedenklichen Salze zusammen mit mindestens einem festen, flüssigen oder halbflüssigen Träger- oder Hilfsstoff in eine geeignete Dosierungsform bringt.

**5.** Pharmazeutische Zubereitung, gekennzeichnet durch einen Gehalt an mindestens einer Verbindung der Formel I nach Anspruch 1 und/oder einem ihrer physiologisch unbedenklichen Salze.

**6.** Verwendung einer Verbindung der Formel I nach Anspruch 1 und/oder eines ihrer physiologisch unbedenklichen Salze zur Herstellung eines Arzneimittels zur Bekämpfung von Krankheiten.

**7.** Verwendung von Verbindung der Formel I und/oder ihrer physiologisch unbedenklichen Salze bei der Bekämpfung von Krankheiten.

Europäisches Patentamt

# EUROPÄISCHER RECHERCHENBERICHT

Nummer der Anmeldung

EP 91 12 1108

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| A,D | EP-A-0 294 647 (MERCK PATENT GMBH)<br>* Ansprüche * | 1-7 | C07D417/04<br>A61K31/54<br>//(C07D417/04,<br>285:00,215:00) |
| A | EP-A-0 180 158 (YOSHITOMI PHARMACEUTICAL INDUSTRIES, LTD.)<br>* Zusammenfassung; Ansprüche * | 1,3,5,6 | |
| A | EP-A-0 303 418 (LABORATOIRE SOBIO S.A.)<br>* Ansprüche 1,7,9,10; Beispiele 35,36,44 * | 1,3,5,6 | |
| A | EP-A-0 381 374 (LES LABORATOIRES BEECHAM S.A.)<br>* Ansprüche 1,7-10 * | 1,3,5,6 | |
| | | | **RECHERCHIERTE SACHGEBIETE (Int. Cl.5 )** |
| | | | C07D |

Der vorliegende Recherchenbericht wurde für alle Patentansprüche erstellt

| Recherchenort | Abschlußdatum der Recherche | Prüfer |
|---|---|---|
| BERLIN | 18 MAERZ 1992 | CHRISTIAN HASS |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)